# FASCICULE DE BREVET EUROPEEN

(11) **EP 1 365 165 B1**
(45) Date de publication et mention de la délivrance du brevet: **18.07.2012**
(21) Numéro de dépôt: 03009662.2
(22) Date de dépôt: 30.04.2003
(51) Int. Cl.: F16D 3/12

(54) **Accouplement conique et prothèse comprenant un tel accouplement**
Kegelkupplung und Prothese, die mit einer solchen Kupplung ausgerüstet ist
Conical coupling and prosthesis comprising such a coupling

(30) Priorité: 22.05.2002 FR 0206196
(43) Date de publication de la demande: 26.11.2003
(73) Titulaire: Tornier, 38330 Saint Ismier (FR)
(72) Inventeur: Hassler, Michel, 38330 Saint Ismier (FR); Real, Cécile, 38000 Grenoble (FR); Pequignot, Jean-Pierre, 06000 Nice (FR); De Mourgues, Philippe, 73290 La Motte Servolex (FR); Allieu, Yves, 34070 Montpellier (FR)
(74) Mandataire: Micheli & Cie SA

(56) Documents cités:
- EP-A- 0 099 167
- EP-A- 0 170 779
- EP-A- 0 420 435
- EP-A- 1 013 242
- EP-A- 1 080 701
- WO-A-02/13730
- WO-A-91/18563
- WO-A-97/11651
- DE-A- 2 933 237
- DE-A- 4 443 051
- FR-A- 2 105 998
- FR-A- 2 580 170
- FR-A- 2 653 660
- GB-A- 2 045 082
- GB-A- 2 319 962
- US-A- 4 997 444

## Description

La présente invention concerne un accouplement conique d'une pièce mâle et d'une pièce femelle, destiné à recevoir notamment des charges de compression axiales. L'accouplement selon l'invention trouve une application particulière, mais non exclusive, dans le domaine des prothèses médicales.

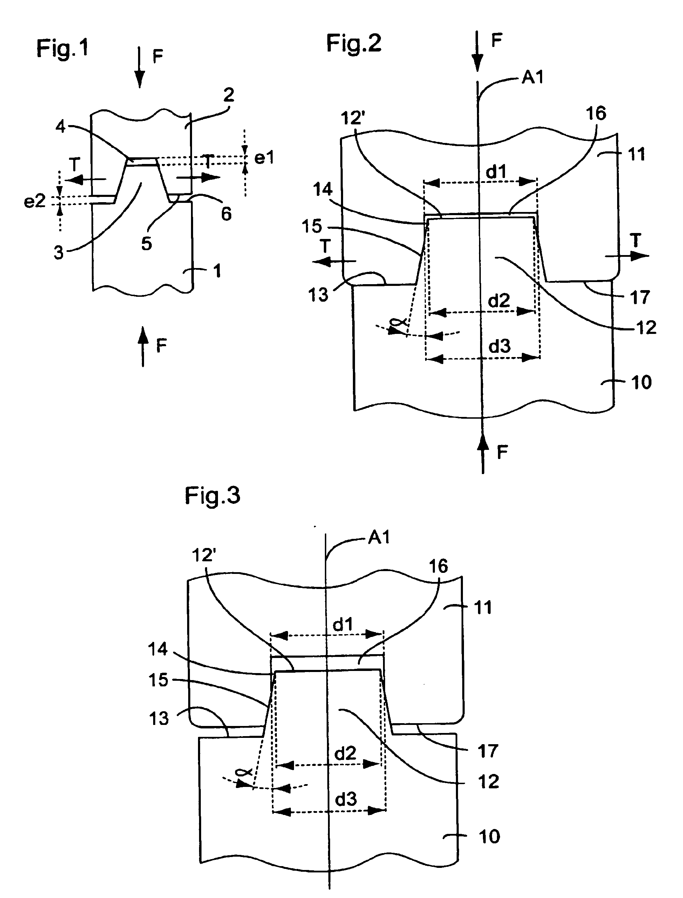

Les accouplements de type conique, tels que les cônes Morse, sont une solution bien connue pour assembler deux pièces de manière simple et démontable. Un accouplement conique est constitué, comme illustré à la figure 1, d'une pièce mâle 1 et d'une pièce femelle 2, généralement métalliques. La pièce mâle 1 comporte un cône 3 emmanché dans un logement conique correspondant 4 de la pièce femelle 2. Les pièces mâle et femelle 1, 2 sont conçues pour que, lorsqu'elles sont en position accouplée, des interstices e1, e2 subsistent entre l'extrémité distale du cône 3 et le fond du logement conique 4 et entre la base, 5, de la pièce femelle 2 et le plan supérieur ou épaulement, 6, de la pièce mâle 1, de manière à obtenir un coincement du cône 3 dans le logement 4 et éviter de rendre l'accouplement hyperstatique. Ces interstices e1, e2 doivent subsister dans toute la plage d'utilisation de l'accouplement. En d'autres termes, les pièces mâle et femelle 1, 2 sont conçues pour résister aux efforts de pénétration de la pièce mâle 1 dans la pièce femelle 2 quelles que soient les charges de compression axiales susceptibles d'être appliquées à l'accouplement.

Ce genre d'accouplement assure un centrage et un blocage en rotation efficaces de la pièce mâle par rapport à la pièce femelle. Toutefois, il nécessite de réaliser la pièce femelle en un matériau très résistant en traction. En effet, en raison de l'angle du cône 3 et de la force de compression axiale nécessaire à la réalisation de l'accouplement, c'est-à-dire à l'assemblage des pièces mâle et femelle, la partie inférieure de la pièce femelle en contact avec le cône 3 subit en permanence des contraintes de traction T, qui augmentent lorsque, en utilisation, l'accouplement est soumis à des charges de compression axiales F.

Aussi, si de tels accouplements sont appropriés pour des pièces mâles et femelles métalliques, et qui ont donc une bonne résistance en traction, leur principe apparaît difficilement transposable à des applications dans lesquelles l'accouplement serait soumis à de fortes charges de compression axiales F, telles que peuvent en recevoir par exemple certaines prothèses, et la matière dans laquelle serait faite la pièce femelle aurait une mauvaise résistance en traction. En pareil cas, en effet, la pièce femelle se romprait au niveau de sa partie inférieure, sous l'action des contraintes de traction T, dès le dépassement de la limite maximale de résistance en traction du matériau constituant la pièce femelle.

On connaît aussi par le document WO 91/18563, figure 6, un accouplement d'une pièce mâle et d'une pièce femelle dans lequel la pièce mâle comprend une partie saillante conique qui est reçue dans un logement conique de la pièce femelle, des moyens auxiliaires d'assemblage de ces pièces mâle et femelle étant prévus sous la forme d'un écrou de compression. Le figure 3 de ce document montre un accouplement selon le préambule de la revendication 1.

La présente invention vise à proposer un accouplement conique dont la pièce femelle est faite en une matière différente de celles généralement utilisées dans les accouplements coniques conventionnels et qui soit capable de limiter les contraintes en traction subies par la pièce femelle même lorsque des charges de compression axiales sont appliquées à l'accouplement.

A cette fin, il est prévu, selon l'invention, un accouplement conique d'une pièce mâle et d'une pièce femelle, la pièce mâle comportant un cône emmanché dans une partie tronconique d'un logement correspondant formé dans la pièce femelle, les pièces mâle et femelle étant bloquées l'une par rapport à l'autre par coincement du cône dans la partie tronconique, caractérisé en ce que la pièce femelle est faite en une matière ayant un module de Young au plus égal à environ 35 GPa et au plus égal à celui de la matière dans laquelle est faite la pièce mâle, et en ce qu'une surface de la pièce mâle perpendiculaire à l'axe de l'accouplement est en contact avec une surface de la pièce femelle également perpendiculaire à l'axe de l'accouplement de sorte que les pièces mâle et femelle sont en butée axiale l'une contre l'autre.

Ainsi, dans l'accouplement selon l'invention, et à la différence des accouplements coniques conventionnels où des interstices sont nécessairement laissés entre la pièce mâle et la pièce femelle, les pièces mâle et femelle sont en butée axiale l'une contre l'autre. L'intensité des contraintes de traction subies par la pièce femelle reste donc constante, et, en particulier, n'augmente pas lorsque l'accouplement est soumis à des charges de compression axiales d'intensité croissante. La pièce femelle peut, par conséquent, être réalisée en un matériau ayant une mauvaise résistance en traction.

Cette solution dans laquelle la liaison entre la pièce mâle et la pièce femelle est volontairement hyperstatique, est rendue possible par l'élasticité intrinsèque de la pièce femelle, ou en d'autres termes par le faible module de Young, inférieur ou égal à environ 35 GPa, de la matière dans laquelle est faite la pièce femelle, et par le fait que la pièce femelle est au moins aussi élastique que la pièce mâle. Grâce à ces propriétés, en effet, la pièce femelle peut, lors de son assemblage avec la pièce mâle, être déplacée axialement vers la pièce mâle le long du cône de cette dernière, par déformation élastique, même après qu'un effet de coincement du cône dans le logement correspondant a été obtenu. En dimensionnant les pièces mâle et femelle pour que la pièce femelle entre en butée axiale contre la pièce mâle avant le dépassement de la limite maximale de résistance en traction de la matière constituant la pièce femelle, l'on peut ainsi obtenir un accouplement conique dans lequel le cône de la pièce mâle est coincé dans le logement de la pièce femelle, et est donc centré et bloqué en rotation dans ce logement, et les contraintes en traction subies par la pièce femelle restent limitées à une valeur fixe inférieure à la limite maximale précitée.

Dans des exemples typiques de réalisation de l'accouplement selon l'invention, la pièce femelle est en pyrocarbone et la pièce mâle est en métal, céramique ou pyrocarbone.

La présente invention propose également une prothèse, par exemple une prothèse de la tête du radius, une prothèse de la tête du cubitus ou une prothèse de hanche, comprenant un accouplement tel que défini ci-dessus, la pièce femelle de l'accouplement constituant une tête de la prothèse et la pièce mâle constituant un col de la prothèse, la prothèse comprenant en outre une queue.

La présente invention vise par ailleurs à proposer un ensemble de pièces mâle et femelle permettant de réaliser l'accouplement tel que défini ci-dessus.

A cette fin, il est prévu, selon l'invention, un ensemble de pièces mâle et femelle, la pièce mâle comportant un cône apte à s'emmancher dans une partie tronconique d'un logement correspondant formé dans la pièce femelle pour s'y coincer et bloquer ainsi les pièces mâle et femelle l'une par rapport à l'autre, caractérisé en ce que la pièce femelle est faite en une matière ayant un module de Young au plus égal à environ 35 GPa et au plus égal à celui de la matière dans laquelle est faite la pièce mâle, en ce que la pièce mâle comporte une surface de butée perpendiculaire à l'axe du cône, et en ce que les pièces mâle et femelle sont dimensionnées pour que, lorsque la pièce mâle et la pièce femelle sont accouplées l'une à l'autre par emmanchement du cône dans la partie tronconique du logement, et sous l'effet d'une charge de compression axiale appliquée à cet accouplement et entraînant un déplacement axial de la pièce femelle par rapport à la pièce mâle par déformation élastique de la pièce femelle après qu'un effet de coincement du cône dans le logement résultant dudit emmanchement a été obtenu, la pièce femelle puisse venir buter contre la surface de butée de la pièce mâle avant que la limite maximale de résistance en traction de la matière dans laquelle est faite la pièce femelle soit dépassée.

D'autres caractéristiques et avantages de la présente invention apparaîtront à la lumière de la description détaillée suivante de plusieurs modes de réalisation de l'invention faite en référence aux dessins annexés dans lesquels :
- la figure 1, déjà commentée, est une vue en coupe d'un accouplement conique selon la technique antérieure ;
- la figure 2 est une vue en coupe d'un accouplement conique selon un premier mode de réalisation de l'invention ;
- la figure 3 est une vue en coupe montrant un état intermédiaire de l'accouplement selon le premier mode de réalisation de l'invention ;
- la figure 4 est une vue en coupe montrant un accouplement conique selon un second mode de réalisation de l'invention ;
- la figure 5 est une vue plane éclatée montrant une prothèse de la tête du radius utilisant l'accouplement selon l'invention ;
- la figure 6 est une vue plane montrant la prothèse de la figure 5 dans un état assemblé ;
- la figure 7 est une vue en coupe d'une cheville à expansion constituant une queue de la prothèse illustrée à la figure 6 ;
- la figure 8 est une vue en coupe montrant la cheville à expansion de la figure 7 insérée dans le radius ;
- la figure 9 est une vue plane montrant une prothèse de la tête du cubitus utilisant l'accouplement selon l'invention ; et
- la figure 10 est une vue plane montrant une prothèse de hanche utilisant l'accouplement selon l'invention.

Dans toute la description suivante, de même que dans les revendications annexées, on entendra par « emmanchement » un ajustement bloqué d'une pièce mâle dans une pièce femelle.

La figure 2 montre un accouplement ou emmanchement conique selon un premier mode de réalisation de l'invention. Cet accouplement comprend une pièce mâle 10 et une pièce femelle 11. La pièce femelle 11 est faite en une matière ayant un module de Young, ou module d'élasticité, inférieur ou égal à environ 35 GPa, par exemple compris entre environ 10 GPa et environ 35 GPa, et inférieur ou égal au module de Young de la matière dans laquelle est faite la pièce mâle 10. A titre d'exemple, la pièce femelle 11 peut être faite en pyrocarbone et la pièce mâle 10 en métal, céramique ou pyrocarbone. De préférence, la matière dans laquelle est faite la pièce femelle 11 a une résistance en compression, exprimée en MPa, plus élevée que sa résistance en traction.

La pièce mâle 10 comporte un cône 12 et un épaulement 13 faisant office de surface de butée perpendiculaire à l'axe, A1, de l'accouplement, c'est-à-dire à l'axe du cône 12. Le cône 12 est coincé dans un logement correspondant 14 de la pièce femelle 11 par emmanchement dans une partie tronconique 15 de ce logement. De préférence, le logement 14 présente, outre la partie tronconique 15 en contact avec le cône 12, une partie de dégagement cylindrique 16 prolongeant la partie tronconique 15 depuis l'extrémité de plus petit diamètre de celle-ci, à proximité du fond du logement 14, et ayant un diamètre d1 supérieur ou égal au diamètre, d2, de l'extrémité distale 12' du cône mâle 12. La partie de dégagement cylindrique 16 sert à recevoir l'extrémité distale 12' du cône mâle 12, comme illustré à la figure 2. Afin de permettre l'insertion de cette extrémité distale 12' du cône mâle 12 dans la partie de dégagement 16, le petit diamètre, d3, de la partie tronconique 15 est également supérieur ou égal au diamètre d2.

L'angle α du cône mâle 12 est sensiblement identique à celui de la partie tronconique 15 du logement 14. Cet angle est choisi suffisamment grand pour que l'emmanchement du cône mâle 12 dans le logement 14 puisse être démonté par l'utilisateur en exerçant un effort raisonnable, et suffisamment petit pour que les pièces mâle et femelle 10, 11 restent fermement bloquées l'une par rapport à l'autre et que la pièce femelle 11 ne se désolidarise pas de la pièce mâle 10 en remontant le long du cône 12 à cause de son élasticité. En pratique, dans le cas notamment d'une pièce femelle conçue en pyrocarbone, l'angle de cône α est typiquement compris entre 2,5 et 5°, et de préférence égal à environ 4°.

La base ou surface d'extrémité proximale, 17, de la pièce femelle 11 est en contact avec l'épaulement 13 de la pièce mâle 10. Ainsi, l'intensité des contraintes de traction T subies par la pièce femelle 11 reste constante, et ceci même lorsque l'accouplement est soumis à une charge de compression axiale F d'intensité variable.

L'accouplement de la figure 2 est obtenu en assemblant la pièce mâle 10 et la pièce femelle 11 de telle sorte que le cône mâle 12 vienne se coincer dans le logement 14 de la pièce femelle 11 pour bloquer les pièces mâle et femelle l'une par rapport à l'autre, comme montré à la figure 3, puis en appliquant une charge de compression axiale supplémentaire pour déplacer axialement la pièce femelle 11 par rapport à la pièce mâle 10 jusqu'à ce que sa base 17 vienne prendre appui sur l'épaulement 13. Lors de ce déplacement axial, la partie inférieure de la pièce femelle 11 dans laquelle est formée le logement 14 s'écarte et subit donc des contraintes de traction d'intensité croissante, en raison de l'angle α du cône, mais ces contraintes de traction n'augmentent plus dès que la pièce femelle 11 arrive au contact de l'épaulement 13. Les dimensions du cône 12 et du logement correspondant 14 sont choisies de façon que la base 17 de la pièce femelle 11 puisse venir buter contre l'épaulement 13 avant que la limite maximale de résistance en traction de la matière constituant la pièce femelle 11 soit dépassée.

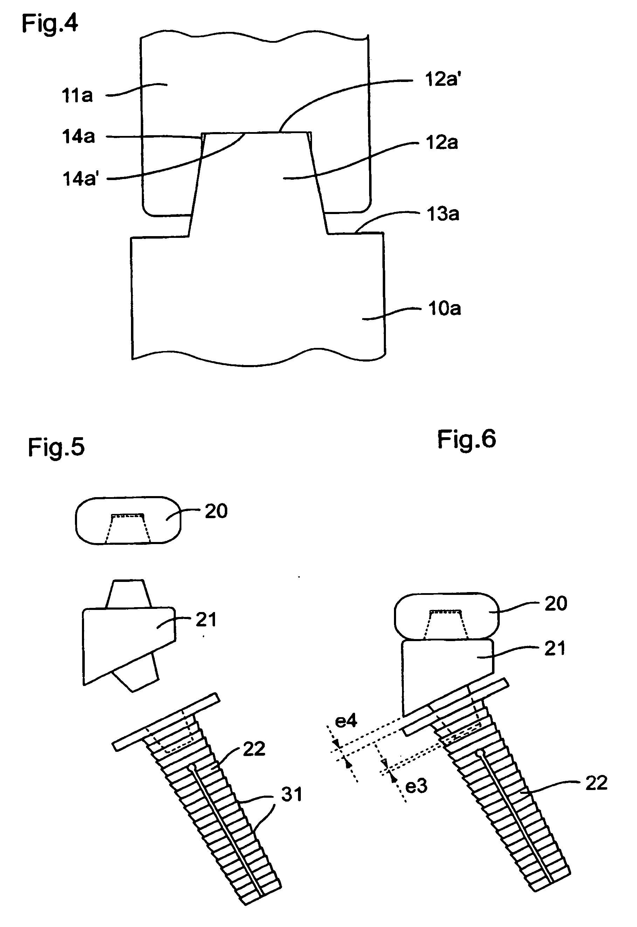

La figure 4 montre un accouplement conique selon un second mode de réalisation de l'invention. Cet accouplement diffère de celui illustré à la figure 2 en ce que la surface de butée de la pièce mâle 10a n'est pas constituée par l'épaulement 13a mais par la surface d'extrémité distale 12a' du cône 12a, laquelle est en contact avec le fond, 14a', du logement 14a de la pièce femelle 11 a. L'accouplement selon ce second mode de réalisation convient mieux à des pièces femelles dont la base est étroite. Il est obtenu de manière comparable à l'accouplement selon le premier mode de réalisation.

Les figures 5 à 10 montrent des prothèses utilisant les principes d'accouplement décrits ci-dessus.

A la figure 5 est représentée une prothèse de la tête du radius. Cette prothèse est constituée d'une tête en pyrocarbone 20, d'un col métallique 21 et d'une queue également métallique 22. Le pyrocarbone constituant la tête 20 est soit massif soit sous la forme d'un revêtement de pyrocarbone recouvrant un substrat en graphite. La tête 20 est destinée à remplacer la tête du radius et la queue 22 à être introduite dans un trou pratiqué dans le radius.

La tête 20 et le col 21 forment ensemble un accouplement du type de celui illustré à la figure 2, la tête 20 jouant le rôle d'une pièce femelle présentant un logement dont au moins une partie est tronconique et le col 21 d'une pièce mâle présentant un cône et un épaulement ou surface de butée contre lequel prend appui la pièce femelle (cf. figure 6). Cet accouplement pourrait toutefois, en variante, être du type de celui illustré à la figure 4.

La queue 22 est couplée au col 21 par une liaison conique conventionnelle, laissant un interstice entre le fond du logement de la pièce femelle, c'est-à-dire la queue 22, et l'extrémité distale du cône de la pièce mâle, c'est-à-dire le col 21, de même qu'entre la base de la pièce femelle et l'épaulement de la pièce mâle (cf. repères e3, e4).

Grâce à cet assemblage modulaire, chaque élément 20, 21, 22 de la prothèse est interchangeable. Chaque élément 20, 21, 22 peut ainsi être conçu en différentes tailles et conserver, quelle que soit sa taille, un logement ou des cônes mâles de formes et de dimensions identiques, de sorte qu'un élément donné de la prothèse puisse être interchangé avec un même élément de taille différente. Il est alors possible pour le chirurgien, une fois choisies les tailles respectives de la tête et de la queue pour un patient donné, de choisir la taille du col de manière à obtenir à un positionnement optimal de la tête par rapport à l'humérus et au cubitus.

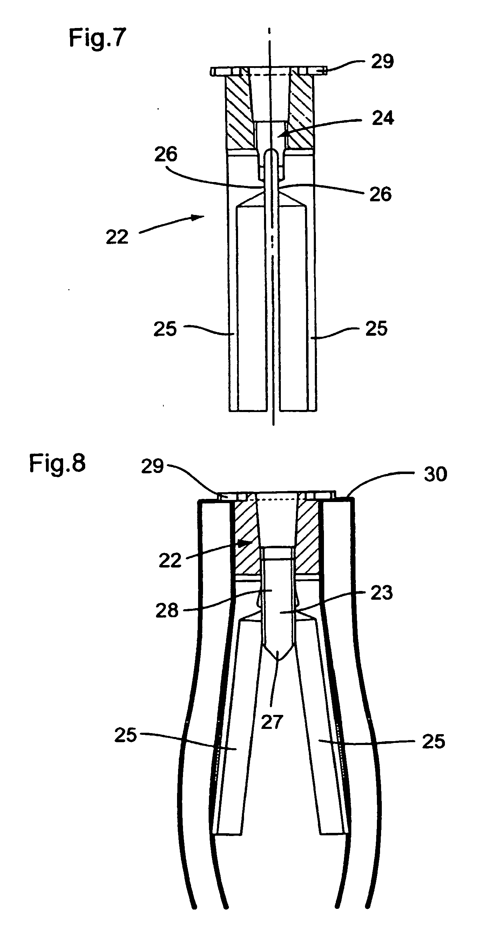

Selon une autre caractéristique de la prothèse de tête de radius selon l'invention, et afin d'assurer un ancrage efficace de la prothèse dans le trou pratiqué dans le radius, la queue 22 se présente avantageusement sous la forme d'une cheville à expansion, apte à se déployer transversalement lorsqu'une vis 23 y est insérée, comme illustré à la figure 8. La queue 22 présente plus particulièrement un passage intérieur 24 ayant une partie taraudée, permettant l'introduction et le vissage de la vis 23, des branches élastiquement déformables 25 constituant la partie déployable de la queue, et des bossages intérieurs 26 aptes à coopérer avec une partie distale conique 27 de la vis 23 pour écarter les branches 25 lors de l'introduction de la vis 23 et avec une partie cylindrique 28 de la vis 23 pour bloquer les branches 25 en position écartée une fois l'introduction de la vis 23 terminée (cf. figures 7, 8). Une collerette 29 est en outre prévue au niveau de l'extrémité proximale de la queue 22, destinée à prendre appui sur une surface réséquée 30 du radius. La surface externe, 31, de la queue 22 est de préférence crantée, comme montré aux figures 5 et 6, de manière à accroître encore l'ancrage de cette queue 22 dans le trou du radius.

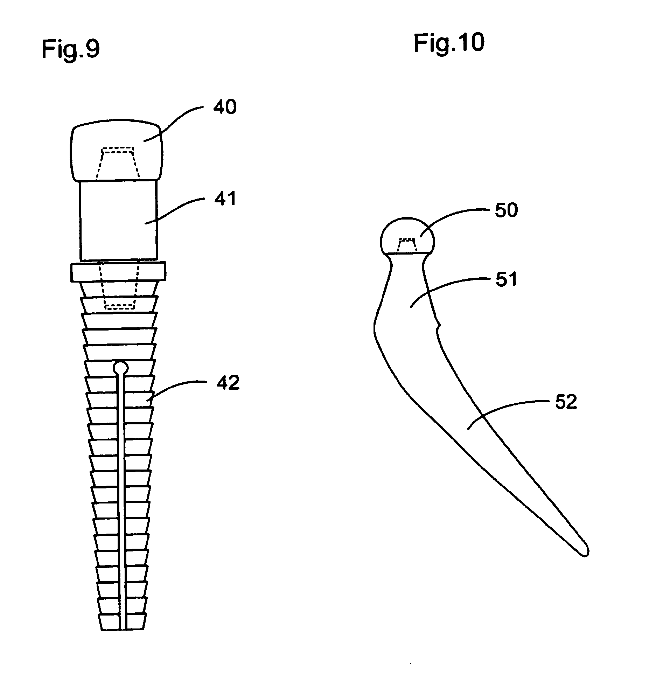

La figure 9 montre une prothèse de la tête du cubitus. Cette prothèse est constituée d'une tête en pyrocarbone 40 destinée à remplacer la tête du cubitus, d'un col métallique 41 et d'une queue 42 également métallique et destinée à être introduite dans un trou pratiqué dans le cubitus. La tête 40 et le col 41 forment un accouplement du type de celui illustré à la figure 2. Cet accouplement pourrait toutefois, en variante, être du type de celui illustré à la figure 4. Le col 41 et la queue 42 sont accouplés l'un à l'autre par un accouplement conique conventionnel.

La figure 10 montre une prothèse de hanche, constituée d'une tête en pyrocarbone 50, d'un col métallique 51 et d'une queue métallique 52. Le col 51 et la queue 52 forment de préférence un ensemble monobloc. La tête 50 et le col 51 forment un accouplement comparable à celui de la figure 2, ou en variante, de celui de la figure 4.

## Revendications

1. Accouplement conique d'une pièce mâle (10) et d'une pièce femelle (11), la pièce mâle (10) comportant un cône (12) emmanché dans une partie tronconique (15) d'un logement correspondant (14) formé dans la pièce femelle (11), les pièces mâle (10) et femelle (11) étant bloquées l'une par rapport à l'autre par coincement du cône (12) dans la partie tronconique (15), **caractérisé en ce que** la pièce femelle (11) est faite en une matière ayant un module de Young au plus égal à environ 35 GPa et au plus égal à celui de la matière dans laquelle est faite la pièce mâle (10), et **en ce qu'**une surface (13) de la pièce mâle perpendiculaire à l'axe (A1) de l'accouplement est en contact avec une surface (17) de la pièce femelle également perpendiculaire à l'axe de l'accouplement de sorte que les pièces mâle et femelle (10, 11) sont en butée axiale l'une contre l'autre.

2. Accouplement selon la revendication 1, **caractérisé en ce que** ladite surface (13) de la pièce mâle perpendiculaire à l'axe de l'accouplement est constituée par un épaulement de la pièce mâle (10) et ladite surface (17) de la pièce femelle perpendiculaire à l'axe de l'accouplement est constituée par une base de la pièce femelle (11).

3. Accouplement selon la revendication 1, **caractérisé en ce que** ladite surface de la pièce mâle (10a) perpendiculaire à l'axe de l'accouplement est constituée par l'extrémité distale (12a') du cône (12a) de la pièce mâle (10a) et ladite surface de la pièce femelle perpendiculaire à l'axe de l'accouplement est constituée par le fond (14a') du logement (14a) de la pièce femelle (11 a).

4. Accouplement selon l'une quelconque des revendications 1 à 3, **caractérisé en ce que** la pièce femelle (11) est en pyrocarbone.

5. Accouplement selon la revendication 4, **caractérisé en ce que** la pièce mâle (10) est en métal, céramique ou pyrocarbone.

6. Accouplement selon l'une quelconque des revendications 1 à 5, **caractérisé en ce que** la matière dans laquelle est faite la pièce femelle (11) a un module de Young compris entre environ 10 GPa et environ 35 GPa.

7. Accouplement selon l'une quelconque des revendications 1 à 6, **caractérisé en ce que** la matière dans laquelle est faite la pièce femelle (11) est plus résistante en compression qu'en traction.

8. Accouplement selon l'une quelconque des revendications 1 à 7, **caractérisé en ce que** le cône (12) de la pièce mâle (10) et la partie tronconique (15) du logement (14) de la pièce femelle (11) ont sensiblement le même angle de cône (α), lequel est compris entre environ 2,5° et environ 5°.

9. Accouplement selon l'une quelconque des revendications 1 à 8, **caractérisé en ce que** la pièce mâle (10) et la pièce femelle (11) sont des pièces d'une prothèse.

10. Prothèse comprenant un accouplement selon l'une quelconque des revendications 1 à 8, la pièce femelle de l'accouplement constituant une tête (20) de la prothèse et la pièce mâle constituant un col (21) de la prothèse, la prothèse comprenant en outre une queue (22).

11. Prothèse selon la revendication 10, **caractérisée en ce que** la queue (22) est couplée au col (21) par un accouplement conique.

12. Prothèse selon la revendication 10 ou 11, **caractérisée en ce que** la queue (22) est sous la forme d'une cheville à expansion.

13. Prothèse selon la revendication 12, **caractérisée en ce que** la surface externe (31) de la queue (22) est crantée.

14. Prothèse selon l'une quelconque des revendications 10 à 13, **caractérisée en ce qu'**elle consiste en une prothèse de la tête du radius (20-22), une prothèse de la tête du cubitus (40-42), ou une prothèse de hanche (50-52).

15. Ensemble de pièces mâle et femelle (10, 11), la pièce mâle (10) comportant un cône (12) apte à s'emmancher dans une partie tronconique (15) d'un logement correspondant (14) formé dans la pièce femelle (11) pour s'y coincer et bloquer ainsi les pièces mâle (10) et femelle (11) l'une par rapport à l'autre, **caractérisé en ce que** la pièce femelle (11) est faite en une matière ayant un module de Young au plus égal à environ 35 GPa et au plus égal à celui de la matière dans laquelle est faite la pièce mâle (10), **en ce que** la pièce mâle comporte une surface de butée (13) perpendiculaire à l'axe (A1) du cône (12), et **en ce que** les pièces mâle et femelle sont dimensionnées pour que, lorsque la pièce mâle (10) et la pièce femelle (11) sont accouplées l'une à l'autre par emmanchement du cône (12) dans la partie tronconique (15) du logement (14), et sous l'effet d'une charge de compression axiale appliquée à cet accouplement et entraînant un déplacement axial de la pièce femelle (11) par rapport à la pièce mâle (10) par déformation élastique de la pièce femelle après qu'un effet de coincement du cône (12) dans le logement (14) résultant dudit emmanchement a été obtenu, la pièce femelle (11) puisse venir buter contre la surface de butée (13) de la pièce mâle (10) avant que la limite maximale de résistance en traction de la matière dans laquelle est faite la pièce femelle (11) soit dépassée.

16. Ensemble de pièces mâle et femelle selon la revendication 15, **caractérisé en ce que** le petit diamètre (d3) de la partie tronconique (15) du logement (14) de la pièce femelle (11) est au moins égal au diamètre (d2) de l'extrémité distale (12') du cône (12) de la pièce mâle (10) et **en ce que** la partie tronconique (15) du logement (14) de la pièce femelle (11) est prolongée au niveau de son extrémité de plus petit diamètre (d3) par une partie de dégagement (16) de diamètre (d1) au moins égal au diamètre (d2) de l'extrémité distale (12') du cône (12) de la pièce mâle (10) et destinée à recevoir cette extrémité distale (12') lorsque la pièce femelle (11) est en butée contre la surface de butée (13) de la pièce mâle (10).

17. Ensemble de pièces mâle et femelle selon la revendication 15 ou 16, **caractérisé en ce que** la pièce femelle (11) est en pyrocarbone et la pièce mâle (10) est en métal, céramique ou pyrocarbone.

18. Ensemble de pièces mâle et femelle selon l'une quelconque des revendications 15 à 17, **caractérisé en ce que** la matière dans laquelle est faite la pièce femelle (11) a un module de Young compris entre environ 10 GPa et environ 35 GPa.

19. Ensemble de pièces mâle et femelle selon l'une quelconque des revendications 15 à 18, **caractérisé en ce que** la matière dans laquelle est faite la pièce femelle (11) est plus résistante en compression qu'en traction.

20. Ensemble de pièces mâle et femelle selon l'une quelconque des revendications 15 à 19, **caractérisé en ce que** le cône (12) de la pièce mâle (10) et la partie tronconique (15) du logement (14) de la pièce femelle (11) ont sensiblement le même angle de cône (α), lequel est compris entre environ 2,5° et environ 5°.

21. Ensemble de pièces mâle et femelle selon la revendication 20, **caractérisé en ce que** l'angle de cône (α) est d'environ 4°.

## Claims

1. Conical coupling of a male member (10) and a female member (11), the male member (10) comprising a cone (12) fitted in a truncated conical portion (15) of a corresponding recess (14) formed in the female member (11), the male and female members (10, 11) being blocked relative to each other by the cone (12) being wedged in the truncated conical portion (15), **characterised in that** the female member (11) is made of a material having a Young's modulus at most equal to about 35 GPa and at most equal to that of the material from which the male member (10) is made, and **in that** a surface (13) of the male member perpendicular to the axis (A1) of the coupling is in contact with a surface (17) of the female member also perpendicular to the axis of the coupling such that the male and female members (10, 11) are in axial abutment against each other.

2. Coupling according to claim 1, **characterised in that** said surface (13) of the male member perpendicular to the axis of the coupling is constituted by a shoulder of the male member (10) and said surface (17) of the female member perpendicular to the axis of the coupling is constituted by a base of the female member (11).

3. Coupling according to claim 1, **characterised in that** said surface of the male member (10a) perpendicular to the axis of the coupling is constituted by the distal end (12a') of the cone (12a) of the male member (10a) and said surface of the female member perpendicular to the axis of the coupling is constituted by the bottom (14a') of the recess (14a) of the female member (11 a).

4. Coupling according to any one of claims 1 to 3, **characterised in that** the female member (11) is of pyrocarbon.

5. Coupling according to claim 4, **characterised in that** the male member (10) is of metal, ceramic or pyrocarbon.

6. Coupling according to any one of claims 1 to 5, **characterised in that** the material from which the female member (11) is made has a Young's modulus comprised between about 10 GPa and about 35 GPa.

7. Coupling according to any one of claims 1 to 6, **characterised in that** the material from which the female member (11) is made is more resistant in compression than in tension.

8. Coupling according to any one of claims 1 to 7, **characterised in that** the cone (12) of the male member (10) and the truncated conical portion (15) of the recess (14) of the female member (11) have substantially the same cone angle (α), which is comprised between about 2.5° and about 5°.

9. Coupling according to any one of claims 1 to 8, **characterised in that** the male member (10) and the female member (11) are pieces of a prosthesis.

10. Prosthesis comprising a coupling according to any one of claims 1 to 8, the female member of the coupling constituting a head (20) of the prosthesis and the male member constituting a neck (21) of the prosthesis, the prosthesis comprising moreover a tail (22).

11. Prosthesis according to claim 10, **characterised in that** the tail (22) is coupled to the neck (21) by a conical coupling.

12. Prosthesis according to claim 10 or 11, **characterised in that** the tail (22) is in the form of an expansible pin.

13. Prosthesis according to claim 12, **characterised in that** the external surface (31) of the tail (22) is notched.

14. Prosthesis according to any one of claims 10 to 13, **characterised in that** it consists of a prosthesis of the head of the radius (20-22), a prosthesis of the head of the cubitus (40-42), or a hip prosthesis (50-52).

15. Set of male and female members (10, 11), the male member (10) comprising a cone (12) adapted to be fitted in a truncated conical portion (15) of a corresponding recess (14) formed in the female member (11) so as to be wedged therein, thus blocking the male and female members (10, 11) relative to each other, **characterised in that** the female member (11) is made of a material having a Young's modulus at most equal to about 35 GPa and at most equal to that of the material from which the male member (10) is made, **in that** the male member comprises an abutment surface (13) perpendicular to the axis (A1) of the cone (12), and **in that** the male and female members are so dimensioned that, when the male member (10) and the female member (11) are coupled to each other by fitting of the cone (12) in the truncated conical portion (15) of the recess (14), and under the effect of an axial compression load applied to this coupling and giving rise to axial displacement of the female member (11) relative to the male member (10) by resilient deformation of the female member after an effect of wedging of the cone (12) in the recess (14) resulting from said fitting has been obtained, the female member (11) can come into abutment against the abutment surface (13) of the male member (10) before the maximum tensile strength limit of the material from which the female member (11) is made is exceeded.

16. Set of male and female members according to claim 15, **characterised in that** the smaller diameter (d3) of the truncated conical portion (15) of the recess (14) of the female member (11) is at least equal to the diameter (d2) of the distal end (12') of the cone (12) of the male member (10) and **in that** the truncated conical portion (15) of the recess (14) of the female member (11) is prolonged at its smaller diameter end (d3) by a clearance portion (16) of a diameter (d1) at least equal to the diameter (d2) of the distal end (12') of the cone (12) of the male member (10) and intended to receive this distal end (12') when the female member (11) is in abutment against the abutment surface (13) of the male member (10).

17. Set of male and female members according to claim 15 or 16, **characterised in that** the female member (11) is of pyrocarbon and the male member (10) is of metal, ceramic or pyrocarbon.

18. Set of male and female members according to any one of claims 15 to 17, **characterised in that** the material from which the female member (11) is made has a Young's modulus comprised between about 10 GPa and about 35 GPa.

19. Set of male and female members according to any one of claims 15 to 18, **characterised in that** the material from which the female member (11) is made is stronger in compression than in tension.

20. Set of male and female members according to any one of claims 15 to 19, **characterised in that** the cone (12) of the male member (10) and the truncated conical portion (15) of the recess (14) of the female member (11) have substantially the same cone angle (α), which is comprised between about 2.5° and about 5°.

21. Set of male and female members according to claim 20, **characterised in that** the cone angle (α) is about 4°.

## Patentansprüche

1. Konische Kupplung aus einem männlichen Teil (10) und einem weiblichen Teil (11), wobei das männliche Teil (10) einen Kegel (12) umfasst, der in einen kegelstumpfförmigen Teil (15) einer entsprechenden Aufnahme (14) eingesetzt ist, die in dem weiblichen Teil (11) ausgebildet ist, wobei das männliche Teil (10) und das weibliche Teil (11) durch Verkeilen des Kegels (12) in dem kegelstumpfförmigen Teil (15) miteinander arretiert werden, **dadurch gekennzeichnet, dass** das weibliche Teil (11) aus einem Material hergestellt ist, das einen Elastizitätsmodul von höchstens gleich ungefähr 35 GPa und höchstens gleich dem des Materials, aus dem das männliche Teil (10) hergestellt ist, aufweist, und dass eine Fläche (13) des männlichen Teils, die senkrecht zu der Kupplungsachse (A1) ist, mit einer Fläche (17) des weiblichen Teils, die ebenfalls senkrecht zu der Kupplungsachse ist, derart in Kontakt steht, dass das männliche Teil und das weibliche Teil (10, 11) axial aufeinander aufliegen.

2. Kupplung nach Anspruch 1, **dadurch gekennzeichnet, dass** die Fläche (13) des männlichen Teils, die senkrecht zu der Kupplungsachse ist, von einer Schulter des männlichen Teils (10) gebildet wird und die Fläche (17) des weiblichen Teils, die senkrecht zu der Kupplungsachse ist, von einer Basis des weiblichen Teils (11) gebildet wird.

3. Kupplung nach Anspruch 1, **dadurch gekennzeichnet, dass** die Fläche des männlichen Teils (10a), die senkrecht zu der Kupplungsachse ist, von dem distalen Ende (12a') des Kegels (12a) des männlichen Teils (10a) gebildet wird und die Fläche des weiblichen Teils, die senkrecht zu der Kupplungsachse ist, von dem Boden (14a') der Aufnahme (14a) des weiblichen Teils (11a) gebildet wird.

4. Kupplung nach einem der Ansprüche 1 bis 3, **dadurch gekennzeichnet, dass** das weibliche Teil (11) aus Pyrokohlenstoff besteht.

5. Kupplung nach Anspruch 4, **dadurch gekennzeichnet, dass** das männliche Teil (10) aus Metall, Keramik oder Pyrokohlenstoff besteht.

6. Kupplung nach einem der Ansprüche 1 bis 5, **dadurch gekennzeichnet, dass** das Material, aus dem das weibliche Teil (11) hergestellt ist, einen Elastizitätsmodul zwischen ungefähr 10 GPa und ungefähr 35 GPa aufweist.

7. Kupplung nach einem der Ansprüche 1 bis 6, **dadurch gekennzeichnet, dass** das Material, aus dem das weibliche Teil (11) hergestellt ist, eine höhere Druckfestigkeit als Zugfestigkeit aufweist.

8. Kupplung nach einem der Ansprüche 1 bis 7, **dadurch gekennzeichnet, dass** der Kegel (12) des männlichen Teils (10) und der kegelstumpfförmige Teil (15) der Aufnahme (14) des weiblichen Teils (11) im Wesentlichen den gleichen Kegelwinkel (α) aufweisen, der zwischen ungefähr 2,5° und ungefähr 5° liegt.

9. Kupplung nach einem der Ansprüche 1 bis 8, **dadurch gekennzeichnet, dass** das männliche Teil (10) und das weibliche Teil (11) Teile einer Prothese sind.

10. Prothese, die eine Kupplung nach einem der Ansprüche 1 bis 8 umfasst, wobei das weibliche Teil der Kupplung einen Kopf (20) der Prothese bildet und das männliche Teil einen Kragen (21) der Prothese bildet, wobei die Prothese außerdem einen Schaft (22) umfasst.

11. Prothese nach Anspruch 10, **dadurch gekennzeichnet, dass** der Schaft (22) durch eine konische Kupplung an den Kragen (21) gekuppelt ist.

12. Prothese nach Anspruch 10 oder 11, **dadurch gekennzeichnet, dass** der Schaft (22) in der Form eines Spreizdübels ist.

13. Prothese nach Anspruch 12, **dadurch gekennzeichnet, dass** die Außenfläche (31) des Schafts (22) eingekerbt ist.

14. Prothese nach einem der Ansprüche 10 bis 13, **dadurch gekennzeichnet, dass** sie in einer Radiusköpfchenprothese (20 - 22), einer Ellenkopfprothese (40 - 42) oder einer Hüftprothese (50 - 52) besteht.

15. Einheit aus einem männlichen Teil und einem weiblichen Teil (10, 11), wobei das männliche Teil (10) einen Kegel (12) umfasst, der dazu geeignet ist, in einen kegelstumpfförmigen Teil (15) einer entsprechenden Aufnahme (14) des weiblichen Teils (11) eingesetzt und verkeilt zu werden, um so das männliche Teil (10) und das weibliche Teil (11) miteinander zu arretieren, **dadurch gekennzeichnet, dass** das weibliche Teil (11) aus einem Material hergestellt ist, das einen Elastizitätsmodul von höchstens gleich ungefähr 35 GPa und höchstens gleich dem des Materials, aus dem das männliche Teil (10) hergestellt ist, aufweist, dass das männliche Teil eine Auflagefläche (13) umfasst, die senkrecht zu der Achse (A1) des Kegels (12) ist, und dass das männliche Teil und das weibliche Teil derart dimensioniert sind, dass, wenn das männliche Teil (10) und das weibliche Teil (11) durch Einsetzen des Kegels (12) in den kegelstumpfförmigen Teil (15) der Aufnahme (14) aneinander gekuppelt sind und unter Einwirkung einer axialen Drucklast, die auf die Kupplung angewendet wird und die eine axiale Verlagerung des weiblichen Teils (11) im Verhältnis zu dem männlichen Teil (10) durch elastische Verformung des weiblichen Teils mit sich bringt, nach einer Einwirkung des Verkeilens des Kegels (12) in der Aufnahme (14), die daraus resultiert, dass das Einsetzen erzielt wurde, das weibliche Teil (11) auf der Auflagefläche (13) des männlichen Teils (10) aufzuliegen kommen kann, bevor die maximale Zugfestigkeit des Materials, aus dem das weibliche Teil (11) hergestellt ist, überschritten wird.

16. Einheit aus einem männlichen Teil und einem weiblichen Teil nach Anspruch 15, **dadurch gekennzeichnet, dass** der kleine Durchmesser (d3) des kegelstumpfförmigen Teils (15) der Aufnahme (14) des weiblichen Teils (11) mindestens gleich dem Durchmesser (d2) des distalen Endes (12') des Kegels (12) des männlichen Teils (10) ist und dass der kegelstumpfförmige Teil (15) der Aufnahme (14) des weiblichen Teils (11) auf Höhe seines Endes mit dem kleineren Durchmesser (d3) durch einen Freigabeteil (16) mit einem Durchmesser (d1) verlängert ist, wobei der Durchmesser (d1) mindestens gleich dem Durchmesser (d2) des distalen Endes (12') des Kegels (12) des männlichen Teils (10) ist, wobei das Freigabeteil (16) vorgesehen ist, dieses distale Ende (12') aufzunehmen, wenn das weibliche Teil (11) auf der Auflagefläche (13) des männlichen Teils (10) aufliegt.

17. Einheit aus einem männlichen Teil und einem weiblichen Teil nach Anspruch 15 oder 16, **dadurch gekennzeichnet, dass** das weibliche Teil (11) aus Pyrokohlenstoff besteht und das männliche Teil (10) aus Metall, Keramik oder Pyrokohlenstoff besteht.

18. Einheit aus einem männlichen Teil und einem weiblichen Teil nach einem der Ansprüche 15 bis 17, **dadurch gekennzeichnet, dass** das Material, aus dem das weibliche Teil (11) hergestellt ist, einen Elastizitätsmodul zwischen ungefähr 10 GPa und ungefähr 35 GPa aufweist.

19. Einheit aus einem männlichen Teil und einem weiblichen Teil nach einem der Ansprüche 15 bis 18, **dadurch gekennzeichnet, dass** das Material, aus dem das weibliche Teil (11) hergestellt ist, eine höhere Druckfestigkeit als Zugfestigkeit aufweist.

20. Einheit aus einem männlichen Teil und einem weiblichen Teil nach einem der Ansprüche 15 bis 19, **dadurch gekennzeichnet, dass** der Kegel (12) des männlichen Teils (10) und der kegelstumpfförmige Teil (15) der Aufnahme (14) des weiblichen Teils (11) im Wesentlichen den gleichen Kegelwinkel (α) aufweisen, der zwischen ungefähr 2,5° und ungefähr 5° liegt.

21. Einheit aus einem männlichen Teil und einem weiblichen Teil nach Anspruch 20, **dadurch gekennzeichnet, dass** der Kegelwinkel (α) ungefähr 4° beträgt.
